# EUROPEAN PATENT APPLICATION

(11) **EP 4 269 609 A1**
(43) Date of publication of application: **01.11.2023**
(21) Application number: 22170479.4
(22) Date of filing: 28.04.2022
(51) Int. Cl.: C12Q 1/6806, C12Q 1/6883, C12Q 1/6886

(54) **METHOD FOR MAKING A GENETIC DETERMINATION BASED ON A HAIR ROOT SAMPLE**

(71) Applicant: Eberhard Karls Universität Tübingen Medizinische Fakultät, 72074 Tübingen (DE)
(72) Inventor: Achberger, Kevin, 72070 Tübingen (DE); Liebau, Stefan, 72414 Rangendingen (DE); Ossowski, Stephan, 70184 Stuttgart (DE); Buena Atienza, Elena, 72076 Tübingen (DE); Admard, Jakob, 72076 Tübingen (DE); Gross, Caspar, 72072 Tübingen (DE)
(74) Representative: Witte, Weller & Partner Patentanwälte mbB

(57) **Abstract**

The present invention relates to a method for making a genetic determination based on a hair root sample, a kit adapted for carrying out said method, and to a use of a hair root sample from a test individual for making a genetic determination.

## Description

The present invention relates to a method for making a genetic determination based on a hair root sample, a kit adapted for carrying out said method, and to a use of a hair root sample from a test individual for making a genetic determination.

### FIELD OF THE INVENTION

The present invention relates generally to the field of molecular biology. More particularly, it concerns methods for making a genetic determination based on a biological sample.

### BACKGROUND OF THE INVENTION

Biological samples, e.g. patient samples, are regularly used to make diagnoses, predictions or other statements related to genetic phenomena. Typically, the nucleic acid contained in the biological sample is used to make these statements or determinations.

In case of using the genome or DNA of the individual to be tested the problem arises that post-transcriptional phenomena are not or not sufficiently detected any may, therefore, falsify the result of the method and relativize the significance of such approaches.

The use of RNA in so-called gene expression analyses addresses this problem by detecting changes in transcription or post-transcriptional processes. However, the expression pattern depends on the type of tissue in the biological sample and the cells that make it up. Cells typically express only a very limited set of genes, namely those required for the respective functions of the cells. Conclusions about the genetic profile of the organism as a whole or about disease-relevant changes in the genome, e.g. only affecting certain genes that are not expressed in the analyzed biological sample, can only be drawn very poorly or not at all using this approach.

Furthermore, the quality of DNA and RNA in commonly used blood samples rapidly decrease which requires the use of stabilizing systems, such as PAXgene^{®}, which cause additional costs and organizational effort. In addition, blood samples have very high levels of globin mRNA which reduces sequencing yield for the genes of interest.

In addition, current methods of obtaining tissue for gene expression analysis are invasive, and include venipuncture and surgical biopsies. Risks to subjects undergoing such procedures include trauma, hematomas, infection, and death.

WO 2005/121374 discloses a method for determining gene expression where the RNA is isolated from a hair follicle. In the known method, the analysis of RNA obtained from hair follicles is performed by means of a microarray. However, the known method only allows RNA quantification, but not analytical examination of the nucleic acid, which would, however, be necessary for the detection of genetic changes or rare genetic variants.

It is therefore the problem underlying the invention to provide a method for genetic determination starting from a biological sample, in which the disadvantages from the prior art are avoided or at least reduced. In particular, a method is to be provided which enables a reliable statement to be made about genetic phenomena, such as, for example, the predisposition to the development of a rare genetic disease or cancer, and which is less stressful and as harmless as possible for the test subject.

The present invention satisfies these and other needs.

### SUMMARY OF THE INVENTION

The problem underlying the invention is solved by the provision of a method for making a genetic determination comprising the following steps:
a) providing a hair root sample from a test individual,
b) isolating nucleic acid from said hair root sample,
c) determining the nucleotide sequence of said nucleic acid to obtain a test nucleotide sequence,
d) comparing said test nucleotide sequence with a reference nucleotide sequence,
e) making a genetic determination depending on the comparison of the test nucleotide sequence with the reference nucleotide sequence.

The problem is also solved by the use of a hair root sample from a test individual for making a genetic determination based on nucleotide sequencing of nucleic acid isolated from said hair root sample.

According to the invention a "hair root sample" is a biological sample comprising a hair root or keratinocytes comprised by or derived from a hair root. In an embodiment of the invention said hair root sample is a hair obtained from the test individual, such as a human or animal being. In a further embodiment one single hair or hair root is sufficient, while 2, 3, 4, 5, 6, 7, 8, 9, or 10 individual hairs or hair roots of the test individual are preferred. Advantageously, the hairs or hair roots can be obtained from the test individual by non-invasive methods, e.g. by hair plucking. The hair root sample can also be obtained from a deep-frozen and subsequently thawed or cooled hair root sample, e.g. stored in a biobank. Unlike other tissue samples, such as blood samples, hair root samples are much more stable and can therefore be stored for longer periods of time, even at room temperature, and can be easily frozen and/or stored in biobanks for longer periods of time. Hair root samples are much more space efficient due to their much smaller size compared to other biological samples, making them easier to store less expensively.

According to the invention, "nucleic acid" includes any kind of nucleic acid material, such as ribonucleic acid (RNA) and deoxyribonucleic acid (DNA). Said nucleic acid is isolated from the hair root sample by methods known to the skilled artisan.

"Determining the nucleotide sequence" or synonymously "sequencing" recited in step (c) of the method according to the invention is to be understood as elucidating the nucleotide sequence of the isolated nucleic acid, either the entire isolated nucleic acid (e.g. whole genome or whole transcriptome), or a section thereof (i.e. sequences of interest, for DNA e.g. whole exome or panels of any number of genes derived by oligo-hybridization or amplicon based methods; for RNA e.g. parts of the transcriptome such as polyA-mRNA, microRNAs, total RNA etc.) or, alternatively, the entire isolated nucleic acid. Said sequencing is carried out by methods well known in the art, including long-read (e.g. by using PacBio SMRT und ONT Nanopore systems) and short-read DNA and RNA sequencing (i.e. by using Illumina sequencing-by-synthesis systems such as NovaSeq). Furthermore, for the very first time high throughput RNA and DNA sequencing can be used in the method according to the invention, even simultaneously for the same sample.

The nucleotide sequence obtained from the hair root sample of the test individual by sequencing or determining the nucleotide sequence is referred to as "test nucleotide sequence".

The "comparison" of said test nucleotide sequence with a reference sequence as carried out in step (d) of the method according to the invention refers to a common alignment procedure carried out to detect nucleotide matches and mismatches of the aligned sequences.

A "reference nucleotide sequence" is a nucleotide sequence not derived from the nucleic acid isolated from the said hair root sample but to a nucleotide sequence derived from a nucleic acid of other origin, e.g., in an embodiment, a nucleic acid from one or multiple other individuals such as an individual suffering from a genetic disease or a healthy reference individual, the two parents of a child, or a child individual of a putative father. In another embodiment said "reference nucleotide sequence" is a nucleotide sequence derived from the human reference genome, e.g. GRCh38 as released from the Genome Reference Consortium.

According to the invention the "genetic determination" as recited in step (e) of the method according to the invention refers to the provision of a result of prognostic value, including a diagnosis, a prognosis, a predisposition information, especially a disease predisposition information, a paternity information etc.

The problem underlying the invention is herewith fully solved.

The inventors have realized that a hair root sample is the ideal source of genetic material that allows a reliable genetic determination, such as the diagnosis of a genetic disease. According to the findings of the inventors, in contrast to other samples, such as blood or saliva samples, much more disease-relevant genes are expressed in a hair root sample or keratinocytes derived therefrom. As it has been found by the inventors, in hair root samples or keratinocytes derived therefrom about 25 % more disease-relevant genes are expressed than in blood samples. About 5 % of the known disease-relevant genes are only expressed in a hair root sample or keratinocytes derived therefrom but not in blood cells or fibroblasts. The method according to the invention thus allows the corresponding diagnoses to be made on several hundred genes which are not expressed, for example, in peripheral blood mononuclear cells (PBMCs) and fibroblasts used in the prior art.

Furthermore, a hair root sample can be easily obtained in a non-invasive manner, e.g. by hair plucking, and does not require blood collection or the use of a skin punch. By simply taking a hair sample, the method according to the invention is suitable for direct use by consumers, whereby no qualified physician is required. This also allows lifestyle applications that previously required a physician for sample collection.

In addition, hair root samples have advantages in biobanking over other samples like blood samples due to their small size.

Further, hair root samples can be stabilized for longer periods of time for transport even under unfavorable conditions and do not require refrigeration, nor are they susceptible to shock such as, e.g., blood samples.

Finally, hair root samples allow the propagation (multiple passengers are possible) of the sample material using *in vitro* keratinocyte culture.

In an embodiment of the invention said nucleic acid is deoxyribonucleic acid (DNA).

Using DNA allows the detection of genetic alterations of the genome. This measure also has the advantage that nucleic acid of high stability is used. Another advantage is that widely common and well-established methods for isolation and sequencing of nucleic acid can be employed. In comparison to DNA obtained from other samples, such as nose or mouth swabs, FFPE material, conserved blood samples etc. the DNA isolated from hair root samples has a significantly higher quality.

In an embodiment of the invention said nucleic acid is ribonucleic acid (RNA).

The use of RNA allows the detection of diseases that cannot be found with classical DNA-based diagnostics. This is due to the fact that in a large number of diseases the genetic alteration is not observed at the DNA level or cannot be interpreted with certainty, but manifests itself at the RNA level during or after transcription. In the case of rare genetic diseases, the causal alteration can currently only be found with DNA-based diagnostics alone in less than 40 % of cases (varies depending on the disease). This embodiment, on the other hand, can find various previously difficult-to-detect causal changes in patients, such as splicing and regulatory variants, loss or gain of gene expression, allelic imbalance and non-sense-mediated decay. Moreover, the use of RNA allows the evaluation of the effect of complex structural variants or the effect of deep-intronic variants that potentially, but rarely, cause splicing defects and are hard to interpret on DNA level.

In another embodiment of the method according to the invention said nucleic acid is RNA and DNA.

Along with the identification of genetic alterations in the genome or the DNA of the test individual the combination of DNA and RNA sequencing additionally allows the detection of splicing and regulatory defects, mono-allelic gene expression, i.e. the loss of expression of one of the two alleles of a gene, 'loss-of-function', i.e. the complete or severe loss of expression of a gene, or overexpression, e.g. due to a local duplication or even chromosomal trisomy. Genetic alterations found on the DNA, e.g. complex structural variants (e.g. identified via short or long nanopore reads), deep intronic variants, etc., can be evaluated or better interpreted by parallel RNA sequencing. It is estimated that up to 20 % additional cases could be detected when using this embodiment, corresponding to 30 million patients worldwide and 3 million patients in the European Union.

In still another embodiment in step (c) of the method according to the invention RNA or/and DNA sequencing is carried out.

This measure has the advantage that well-established methods for determining the nucleotide sequence of said nucleic acid are employed, such as high-throughput sequencing, next generation sequencing (NGS), third generation sequencing (single molecule sequencing, long read sequencing), nanopore sequencing, single cell sequencing, etc.

In an embodiment of the invention said DNA sequencing comprises epigenome sequencing, preferably to obtain information of methylation of said DNA.

An epigenome consists of a record of the chemical changes to the DNA and histone proteins of an organism; these changes can be passed down to an organism's offspring via transgenerational stranded epigenetic inheritance. Changes to the epigenome can result in changes to the structure of chromatin and changes to the function of the genome. The epigenome is involved in regulating gene expression, development, tissue differentiation, and suppression of transposable elements. Unlike the underlying genome, which remains largely static within an individual, the epigenome can be dynamically altered by environmental conditions. This measure has, therefore, the advantage that genetic alterations in the test individual can be determined which are associated with epigenetic phenomena such as the methylation of DNA or acetylation and methylation of histones or accessible, open regions of the chromatin. Epigenome sequencing can be carried out by using methods well-known in the art, such as whole genome bisulfite sequencing (WGBS), targeted, or methyl-capture sequencing, chromatin immunoprecipitation (ChIP) sequencing, DNAsel-seq, ATAC-seq, etc.

In an embodiment of the method according to the invention after step (a) and before step (b) the following step (a') is carried out:
(a') subjecting said hair root sample to a liquid causing a lysis of the cells and the release of the nucleic acid from the cells, and
wherein in step (b) said nucleic acid is isolated from said liquid.

This measure advantageously ensures the release of the nucleic acid for subsequent isolation. Lysis buffers can be used in this embodiment, which are well known to the skilled person.

In yet another embodiment of the method according to the invention after step (a) and before step (b) the following step (a") is carried out:
a") cultivating keratinocytes comprised by said hair root sample, and
wherein in step (b) nucleic acid from said cultivated keratinocytes or descendant cells thereof is isolated.

This measure allows in an advantageous manner the unlimited propagation of the sample material for applications that require very large amounts of DNA or RNA, such as Nanopore or PacBio long-read sequencing. It further allows the detection of various previously difficult-to-detect causal changes in patients, such as splicing and regulatory variants, loss of gene expression, complex structural variants, etc. In addition, keratinocyte cultures can also be obtained from the same cell material, which can be used for the production of induced pluripotent stem cells. These can be used, for example, for personalized medicine, e.g. drug trials on individual patients, or for regenerative medicine or transplantation, respectively.

In still another embodiment of the method according to the invention before step (a) the following step (a⁰) is carried out:
a⁰) obtaining a hair root sample from a test individual by hair plucking.

This measure provides and obtains the hair root sample in a simple, non-invasive manner without the need for a physician or medically trained personnel. The method according to the invention is herewith adapted for direct use by consumers, whereby no qualified physician is required. This also allows lifestyle applications.

In another embodiment of the method according to the invention after step (a) and/or, if applicable, after step (a") the following step (a'") is carried out:
a'") subjecting the hair root sample and/or, if applicable, said cultivated keratinocytes to a metabolome analysis.

The metabolome refers to the complete set of small-molecule chemicals found within a biological sample, such as the hair root sample of the invention. The small molecule chemicals found in a given metabolome may include both endogenous metabolites that are naturally produced by an organism, such as amino acids, organic acids, nucleic acids, fatty acids, amines, sugars, vitamins, co-factors, pigments, antibiotics, etc., as well as exogenous chemicals, such as drugs, environmental contaminants, food additives, toxins and other xenobiotics, that are not naturally produced by an organism. This measure has, therefore, the advantage that, in addition to the sequence analysis, also an analysis of the metabolome of the test individual is carried out. This allows, via the comparison with the metabolome of a reference individual, the detection of metabolic disorders or diseases. A metabolome analysis is performed by using procedures well known in the art. E.g., metabolites can be extracted, optionally with the addition of internal standards and derivatization. During sample analysis, metabolites are quantified, e.g. by liquid chromatography or gas chromatography coupled with MS and/or NMR spectroscopy. The raw output data can be used for metabolite feature extraction and further processed before statistical analysis, such as PCA. Many bioinformatic tools and software are available to identify associations with disease states and outcomes, determine significant correlations, and characterize metabolic signatures with existing biological knowledge.

In a still further embodiment of the method according to the invention after step (b) and before step (c) the following step (b') is carried out:
b') preparing a sequencing library based on said nucleic acid thereby transforming said nucleic acid into a form appropriate for a sequencing technology, and
wherein in step (d) the nucleotide sequence of said sequencing library is determined to obtain said test nucleotide sequence.

With this measure, the technical prerequisites for high-throughput sequencing are created in an advantageous way. In this step the isolated DNA or RNA is brought into a form appropriate for sequencing, e.g. with common sequencing machines based on Sanger sequencing, sequencing-by-synthesis, nanopore sequencing, single molecule real-time sequencing, single-cell sequencing, or other technologies. This can be achieved by using various kits offered by sequencing companies such as Illumina, NEB, Agilent, Pacific Biosciences, ONT etc. Technically, sequencing library preparation steps vary substantially for the different sequencing technologies, but often includes steps such as quality control of the isolated nucleic acids, nucleic acid shearing, reverse transcription (only RNA), adapter ligation, barcoding, amplification by PCR, among others. DNA and RNA isolated from hair roots or keratinocytes can be used with any type of currently available library preparation kits.

In another embodiment of the invention said reference nucleotide sequence is a nucleotide sequence of a disease-associated gene, including a cancer-associated gene, and wherein said genetic determination is a diagnosis of a disease or predisposition to a disease based on the disease-associated gene.

By this measure, in an advantageous manner, the method according to the invention is configured as a diagnostic methods allowing the diagnosis of a genetic or inherited disease or a predisposition to disease or cancer.

In yet another embodiment of the method according to the invention the following additional step (d') is carried out:
d') comparing a nucleotide sequence of a nucleic acid isolated from a non-hair-root sample of the test individual with said test nucleotide sequence.

According to this embodiment, a "non-hair-root sample" is a biological sample containing DNA and/or RNA, which originates from the test individual but which is not said or any hair root sample. In other words, another sample from a different tissue is taken from the test individual in addition to the hair root sample. DNA and/or RNA is also isolated from this additional sample, its nucleotide sequence determined, and, like the nucleotide sequence of the DNA or RNA from the hair root sample, is compared with the reference nucleotide sequence. Preferably, said non-hair-root sample is a tumor sample, e.g. a blood tumor sample, further preferably said blood tumor is selected from the group consisting of acute myeloid leukemia (AML), chronic lymphocytic leukemia (CLL), and chronic myelogenous leukemia (CML).

By this measure the method according to the invention is adapted for cancer diagnostics. Modern tumor diagnostics by next generation sequencing (NGS) require the comparison of DNA sequencing data of the tumor samples and of a healthy tissue (the 'normal sample') from the same patient. This tumor-normal sequencing allows for detection of mutations only affecting the tumor, so-called somatic variants, some of which are responsible for tumor proliferation. In the art NGS diagnostics for solid tumors typically uses a blood sample as healthy control. In contrast, the invention uses the hair-root derived DNA for getting healthy, 'normal' or control DNA which is of higher quality and easier to obtain. The hair-root derived DNA is then used as a healthy reference for distinguishing somatic (tumor-specific) mutations from inherited germline variants in the DNA isolated from the tumor sample with the goal of identifying tumor-specific, somatic mutations in cancer-associated genes driving tumor proliferation and informing treatment selection. At the same time the hair-root DNA can be used to analyze germline or inherited genetic variants, respectively, in order to identify cancer risk variants.

In a further embodiment of the method according to the invention said reference nucleotide sequence is a nucleotide sequence of a baldness-associated gene, and wherein said genetic determination is a determination of reasons of baldness.

By this measure the method according to the invention is adapted to allow the elucidation of any genetic reasons of baldness in an advantageous manner.

Another subject-matter of the invention relates to a kit for making a genetic determination, comprising:
- a container for receiving a hair root sample,
- a nucleic acid sequencing buffer solution, and
- a manual for carrying out the method according to the invention, and, optionally,
- a cell lysis buffer solution, and, further optionally,
- a keratinocyte culture medium.

A "kit" is a combination of individual elements useful for carrying out the method of the invention, wherein the elements are optimized for use together in the methods. The kit may also contain additional reagents, chemicals, buffers, reaction vials etc. which may be useful for carrying out the method according to the invention. Such a kit unifies all essential elements required to work the method according to the invention, thus minimizing the risk of errors. Therefore, such kits also allow semi-skilled laboratory staff to perform the method according to the invention.

The container for receiving the hair root sample can be used for storing and/or shipping the hair root sample, e.g. to a remote laboratory for further or additional analyses. In an embodiment of the invention the container for receiving the hair root sample can be realized by the system disclosed in WO 2018/065514.

The features, characteristics, advantages and embodiments disclosed for the method according to the invention apply likewise to the kit according to the invention.

It is to be understood that the before-mentioned features and those to be mentioned in the following cannot only be used in the combination indicated in the respective case, but also in other combinations or in an isolated manner without departing from the scope of the invention.

The invention is now described and explained in further detail by referring to the following non-limiting examples and drawings.

### BRIEF DESCRIPTION OF THE DRAWINGS

- Fig. 1:: Schematics of hair-based culture and DNA/RNA isolation.
- Fig. 2:: Functional annotation of mapped RNA-seq reads obtained from two hair-based keratinocyte cultures.
- Fig. 3:: Functional annotation of mapped RNA-seq reads obtained from nine fresh hair root samples.
- Fig. 4:: Expression of known disease genes in hair-based keratinocyte cultures and fresh hair roots compared to frequently used sample types for clinical diagnostics.
- Fig. 5:: Comparison of OMIM disease genes expressed at confidently detectable levels for diagnostic analysis (>3tpm) in different samples.
- Fig. 6:: Similarity of gene expression patterns in different tissues.
- Fig. 7:: Keratinocytes are the best sampling material for Nanopore-based transcriptome analysis.
- Fig. 8:: Example application for hair root derived RNA in clinical diagnostics of a rare disease patient.
- Fig. 9:: Flowchart illustrating a variant of the method according to the invention for diagnosis of a (rare) genetic disease.
- Fig. 10:: Flowchart illustrating an embodiment of the method according to the invention for diagnosis of blood cancer and selection of targeted therapies.
- Fig. 11:: Flowchart illustrating an embodiment of the method according to the invention for identifying hair loss specific patterns.
- Fig. 12:: Flow chart illustrating an embodiment of the method according to the invention including generic wet-lab library preparation and sequencing workflow.

### EXAMPLES

### 1. Material and methods

### Classic keratinocyte isolation and culture according to Aasen

Keratinocyte isolation and culture referred as "classic" was based on a protocol modified from Aasen et al., Efficient and rapid generation of induced pluripotent stem cells from human keratinocytes, Nat. Biotechnol. 2008 Nov;26(11):1276-84. Briefly, hair with visibly intact outer root sheaths were plucked from donor persons using forceps (Fine Science Tools, German) and were directly placed in DMEM high glucose medium (Thermo Fisher Scientific, USA) + antibiotics-antimycotics (AA, Thermo Fisher Scientific, USA) until further processed. Hair roots with intact outer root sheaths were then cut off from the hair and were placed on T25 flasks (Greiner Bio-One, Germany) which have been coated with matrigel (Corning, USA) which was 1:10 diluted in Epilife (Thermo Fisher Scientific, USA) at 37°C for 60 min. Afterwards, a drop of a 1:5 diluted matrigel was placed on top. After 2 hours in a 37°C incubator, isolation medium (DMEM high glucose + 10-15 % Fetal bovine serum (Thermo Fisher Scientific, USA) + 1 % Non-essential-amino acids (NEAA) + 1 % AA + 10 ng/ml fibroblast growth factor 2 (FGF2, Peprotech, USA) + 10 µM Y-27632 (ROCK-inhibitor, Ascent Scientific, USA)) was added carefully to the flasks and was changed daily until outgrowth of keratinocytes occurred. Then, medium was changed to Epilife +HKGS (Thermo Fisher Scientific, USA) + ROCK inhibitor and cells were changed daily. When confluency was reached, cells were lifted with Dispase (from Bacillus polymyxa, Corning, USA) and either replated or frozen in Synth-A-Freeze (Thermo Fisher Scientific, USA). For replating, well plates were coated with collagen IV (1:100 in Phosphate-Buffered Saline without calcium and magnesium (PBS --), Sigma Aldrich, USA) for 1 h at 37°C.

### RNA/DNA isolation from cultured keratinocytes

For isolation of DNA/RNA, keratinocytes were isolated from hair roots of donor persons using the Aasen method and cultured in Epilife + HKGS on collagen IV-coated well plates until confluency was reached. Keratinocyte medium was then removed and cells were washed once with PBS --. Afterwards 500 µl of TrypLE (Thermo Fisher Scientific, USA) was added to each well and cells were place in a 37°C incubator for 8-20 minutes. Incubation was ended by adding PBS -- when cells appeared fully detached. The cells were then collected in a 15 ml conical tube, centrifuged at 1500 rpm for 2 min and supernatant was discarded. If not directly isolated, cell pellets were frozen at -80°C. For isolation, either DNeasy Blood & Tissue Kit (Qiagen, Germany) for DNA or RNeasy mini kit (Qiagen, Germany) for RNA were used. Kits were used according to the manufacturer's protocol with the following specifications: For DNA extraction, cell pellets were incubated with 280 µl ATL buffer + 28µl Proteinase K (both provided by the DNeasy kit) at 56°C for 10 min. After incubation, 28 µl of RNase I (Stock concentration: 10 µg/ml) was added to each sample and incubated for 3 min at RT. Elution of final DNA was done with 100 µl of AE Buffer provided by the kit. For RNA isolation, 700 µl RLT buffer (provided by the RNeasy mini kit) were added to the cell pellet. The solution was then transferred to a QiaShredder (Qiagen, Germany) column and centrifuged at 13.000 rpm for 1 min. DNAse digestion was performed on the column according to the manufacture's protocol. Final RNA was eluted in 2x the same 30 µl AE (provided by the DNeasy micro kit).

### Direct RNA/DNA isolation from hair

Hairs with intact outer root sheaths dedicated for RNA/DNA extraction were plucked with forceps from 3 donor persons. For RNA isolation, 5 hair roots and for DNA isolation, 10 hair roots per sample were used. To prevent drying, hairs were directly placed in petri filled with DMEM high glucose. Afterwards, hair roots were cut off from the hair shaft and placed in 1.5 ml reaction tubes filled with PBS -- (without calcium/magnesium, Thermo Fisher Scientific, USA). For isolation, either DNeasy Blood& Tissue Kit (Qiagen, Germany for DNA) or RNeasy micro kit (Qiagen, Germany for RNA) were used. Kits were used according to the manufacturer's protocol with the following specifications: For DNA extraction, PBS -- was removed and replaced with 180 µl ATL buffer + 20µl Proteinase K (both provided by the DNeasy kit). Samples were incubated at 56°C for 1 h. During that time, samples were vortexed every 20 min. After incubation, the supernatant (excluding the hairs) was placed in a fresh 1.5 ml reaction tube, 20 µl of RNase I (Stock concentration: 10 µg/ml) was added to each sample and incubated for 3 min at RT. Elution of final DNA was done 2x with the same 20 µl of AE Buffer provided by the kit. For RNA isolation, PBS -- was removed, replaced with 700 µl RLT buffer (provided by the RNeasy micro kit) and tubes were vortexed for 30 seconds. The supernatant was transferred to a QiaShredder (Qiagen, Germany) column and centrifuged at 13.000 rpm for 1 min. DNA digestion was performed on the column according to the manufacture's protocol. Final RNA was eluted 2x in the same 16 µl RNAse-free water (provided by the RNeasy micro kit).

### Short-read RNA sequencing

RNA quality was determined by measuring 260/280 and 230/260 absorbance ratio on a spectrophotometer (Nanodrop ND-1000; Peqlab), RNA concentration using the Qubit Fluorometric Quantitation and RNA Broad-Range Assay (Thermo Fisher Scientific) and RNA Integrity Number RIN using the Fragment Analyzer 5300 and the Fragment Analyzer RNA kit (Agilent Technologies). For library preparation, mRNA fraction was enriched using polyA capture from 100ng of total RNA using the NEBNext Poly(A) mRNA Magnetic Isolation Module (NEB). Next, mRNA libraries were prepared using the NEB Next Ultra II Directional RNA Library Prep Kit for Illumina (NEB) according to the manufacturer's instructions. Library molarity was determined by measuring the library size (approximately 400 bp) using the Fragment Analyzer 5300 and the Fragment Analyzer DNA HS NGS fragment kit (Agilent Technologies) and the library concentration (>0.5 ng/µl) using Qubit Fluorometric Quantitation and dsDNA High Sensitivity assay (Thermo Fisher Scientific). In the first experiment, the libraries were denatured according to the manufacturer's instructions, diluted to 270 pM and sequenced as paired-end 2x100bp reads on an Illumina NovaSeq 6000 (Illumina) with a sequencing depth >25 million clusters per sample.

### Long-read RNA sequencing

RNA extraction and RNA quality control was performed as described above for short-read RNA sequencing. Blood samples in PAXgene Blood RNA Tubes (16x100 mm / 2.5 mL) were mixed gently 10 times and stored overnight at room temperature before freezing. Total RNA was extracted with the PAXgene Blood RNA Kit using QIAsymphony RNA kit (Qiagen). For processing PAXgene Blood RNA samples, 700 ng of RNA was used following the NEBNext^{®} Globin & rRNA Depletion Kit (Human/Mouse/Rat) prior to cDNA preparation. For library preparation of all types of RNA samples, the cDNA-PCR Sequencing (SQK-PCS109) kit was used according to manufacturer's instructions. A total of 50 ng of total RNA was annealed for strand-switching reaction and reverse transcribed with Maxima H Minus RT (Thermo Fisher Scientific). The resulting cDNA was amplified with LongAmp Ta Master Mix (NEB). Library molarity was determined by measuring the library size using the Fragment Analyzer 5300 and the Fragment Analyzer DNA HS NGS fragment kit (Agilent Technologies) and the library concentration (>5 ng/µl) using Qubit Fluorometric Quantitation and dsDNA High sensitivity assay (Thermo Fisher Scientific). The Rapid Adaptors were added to the amplified cDNA and <100 fmol of the library was loaded on a PromethlON flow cell (FLO-PRO002).

### Short-read DNA sequencing

For short-read sequencing the inventors used DNA isolated from cultured keratinocyte cells. DNA quality was determined by measuring 260/280 and 230/260 absorbance ratio on a spectrophotometer (Nanodrop ND-1000; Peqlab). DNA was quantified with the Qubit Fluorometric Quantitation and DNA High-Sensitivity Assay (Thermo Fisher Scientific). Genomic integrity was assessed using pulse-field capillary electrophoresis with the FemtoPulse instrument and the Genomic DNA 165 kb Kit (Agilent). A total of 350 ng of DNA were used as input for the library preparation with the Illumina DNA PCR-Free Prep, Tagmentation protocol according to the manufacturer's instructions (Illumina) on a Biomek i5 Automated Liquid Handling Workstation (Beckman Coulter). The final pool with a molarity of 600 pM was loaded on a NovaSeq S4 flow cell (300 cycles) and sequenced in paired-end mode.

### DNA sequencing long-read

For long-read sequencing the inventors used DNA isolated from cultured keratinocyte cells. DNA quality was determined by measuring 260/280 and 230/260 absorbance ratio on a spectrophotometer (Nanodrop ND-1000; Peqlab). DNA was quantified with the Qubit Fluorometric Quantitation and DNA High-Sensitivity Assay (Thermo Fisher Scientific). Genomic integrity was assessed using pulse-field capillary electrophoresis with the Femto*Pulse* instrument and the Genomic DNA 165 kb Kit (Agilent). A total of 1,5 µg of size selected DNA was used for the 1D library preparation following the protocol of Ligation Sequencing (SQK-LSK109) from Oxford Nanopore Technologies (ONT) upon size selection of 2,5 µg of genomic DNA and clean-up with SPRI beads at a 0.7 ratio when necessary. Two library preparations were performed per sample, and 500-600 ng of each library was loaded on a single flow cell (FLO-PRO002) on a PromethlON instrument (Oxford Nanopore Technologies, ONT).

### Analysis of short-reads RNA-seq data

The inventors used short-read RNA-seq data from hair-based keratinocyte cultures, fresh hair roots, as well as from blood and fibroblasts to estimate the similarity of gene expression profiles of the different tissue types for clinical diagnostics. Read quality of RNA-seq data in fastq-files was assessed using ngs-bits (v.2020_06), which identifies sequencing cycles with low average quality, adaptor contamination, or repetitive sequences from PCR amplification. Reads were aligned using STAR v2.7.3a to the GRCh38 human reference genome. Alignment quality was analyzed using ngs-bits (v.2020_06) and visually inspected in the Integrative Genome Viewer (v2.7.2). Reads overlapping known genes were annotated as originating from CDS-exons, 5'UTR-exon, 3'UTR-exon, introns, intergenic regions or from the vicinity of transcription start sites (TS) or transcription end sites (TES). The fraction of reads aligning to exonic regions is used for RNA-seq quality comparison between samples.

Normalized read counts for all genes were obtained using Subread (v2.0.0) and edgeR (v3.30.3). Genes not showing a minimum gene expression value of 1 cpm (counts per million) in at least 2 samples were removed. The distribution of logarithmized cpm-normalized (log₂(cpm)) expression values shows similar characteristics over all samples.

### Sample similarity and differential gene expression analysis

Using the filtered and normalized expression data. Spearman's rank correlation coefficient was calculated for each pair of samples to measure pairwise similarity. Hierarchical clustering was performed on the resulting similarity values. Differential gene expression analysis was conducted using edgeR. A statistical model incorporating the group property of samples was tested by fitting a negative binomial distribution using a generalized linear model (GLM) approach. For each gene, gene expression fold changes (log2 fold change) were computed and a statistical test was performed to assess the significance, which is given as raw p-value and adjusted p-value (FDR).

### Comparison of expressed disease genes in four tissue types

The inventors compared gene expression values for 4237 disease genes annotated in Online Mendelian Inheritance in Men (OMIM; online catalogue of human genes and genetic disorders, https://www.omim.org) between three tissues that can non-invasively be sampled (and cultured) from patients (blood, fresh hair roots, hair based keratinocyte culture), and additionally of fibroblasts cultured from skin punch biopsies (often used for rare disease diagnostics). Expression values were labeled as low (<0.5 cpm), moderate (0.5 to 3 cpm) and high (>3 cpm). Venn diagrams were used to compare and visualize the number of OMIM disease genes reaching a given level (e.g. high expression) in the four sample types. The inventors defined genes with at least 3cpm as diagnostically accessible using a given tissue type. Based on known associations between phenotypic disease descriptions (Human Phenotype Ontology) and disease genes (OMIM or HGMD) the inventors developed a tool for selecting the optimal tissue type (or combination of tissues) as samples for clinical diagnostics of the disease. The tool is simply calculating the number known disease genes for a given disease that show high expression levels in a given tissue type or combination.

### Analysis of long-read RNA-seq data

Long RNA reads obtained using ONT PromethlON from blood, fresh hair and hair-based keratinocyte cultures was analyzed using an in-house pipeline consisting of quality control, spliced alignment with minimap and transcript isoform detection. Long reads are classified as Full-Splice-Match (FSM) if they completely cover a known transcript isoform, Incomplete-Splice-Match (ISM) if the partly cover a known isoform but show no alternative splicing, Novel-In-Catalog (NIC) if they represent a new isoform of a known gene (new combination of known splice junction, novel splice junctions), and Other if they do not overlap known exons. Assuming that detection of novel isoforms or novel genes is a rare event, the inventors estimated the quality of long read RNA-seq in the compared tissues as the fraction of FSM and ISM reads.

### 2. Results

### Schematics of hair-based culture and DNA/RNA isolation

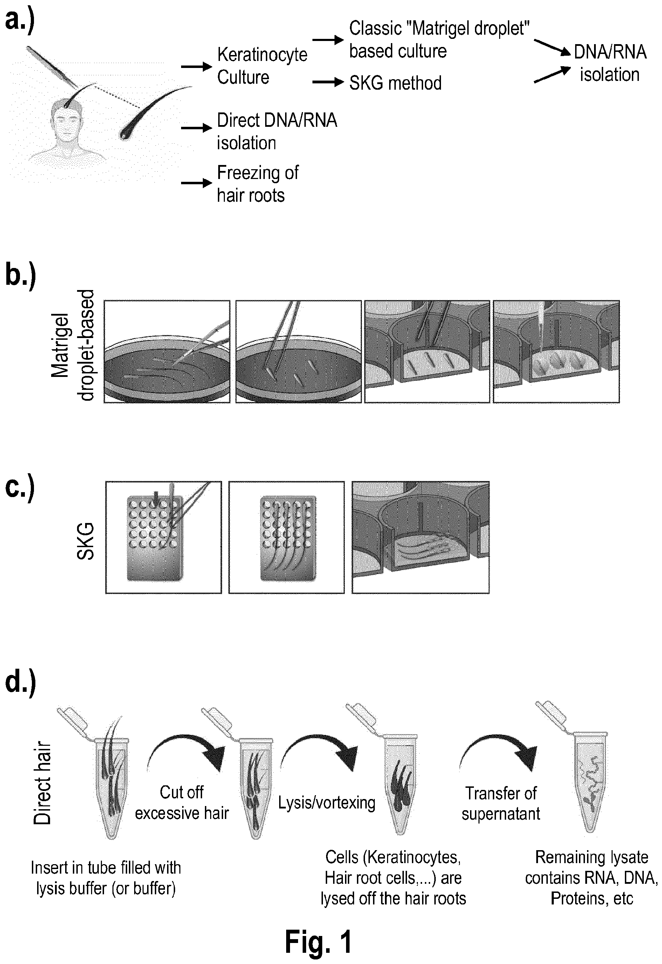

In Fig. 1 a diagram is shown illustrating the hair-based culture and DNA/RNA isolation. a) A patient's DNA and RNA can be obtained from i/ hair-based keratinocyte cultures, ii/ directly from fresh hair roots, or iii/ from fresh-frozen hair roots; b) hair-based keratinocyte culture with matrigel droplets; c) hair-based keratinocyte culture with a system for storing hair samples, e.g., as described in WO 2018/065514; d) direct DNA and RNA extraction from fresh hair roots.

### Functional annotation of mapped RNA-seq reads obtained from two hair-based keratinocyte cultures

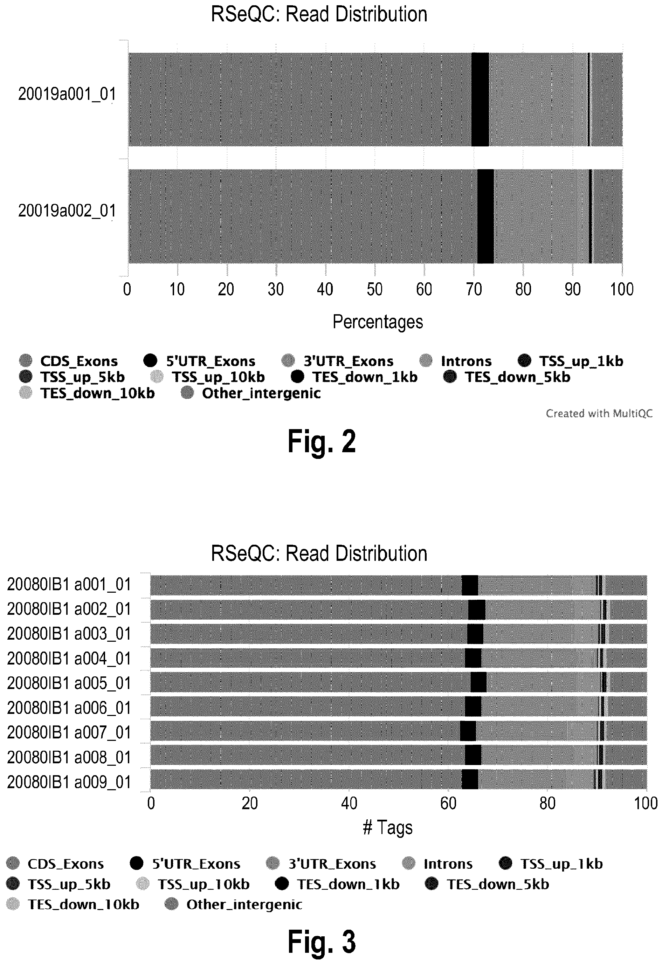

In Fig. 2 the result of a functional annotation of mapped RNA-seq reads obtained from two hair-based keratinocyte cultures is depicted. For benchmarking purposes a large fraction of reads aligned to exonic regions is considered good, while large fractions of intronic or intergenic reads could indicate issue of the wet-lab procedure. More than 93 % of reads align to known genes and more than 90 % originate from exonic regions, indicate a very high quality of the sampling and sequencing procedure.

### Functional annotation of mapped RNA-seq reads obtained from nine fresh hair root samples

The result of a functional annotation of mapped RNA-seq reads obtained from nine fresh hair root samples is shown in Fig. 3. More than 90 % of reads align to known genes and more than 85 % originate from exonic regions, indicating a very high quality of the sampling and sequencing procedure. Quality measures for fresh hair are only marginally lower than measures for hair-based keratinocyte cultures, which could reflect the reduced input material or the more complex cell type composition of hair roots compared to a culture.

### Expression of known disease genes in hair-based keratinocyte cultures and fresh hair roots compared to frequently used sample types for clinical diagnostics

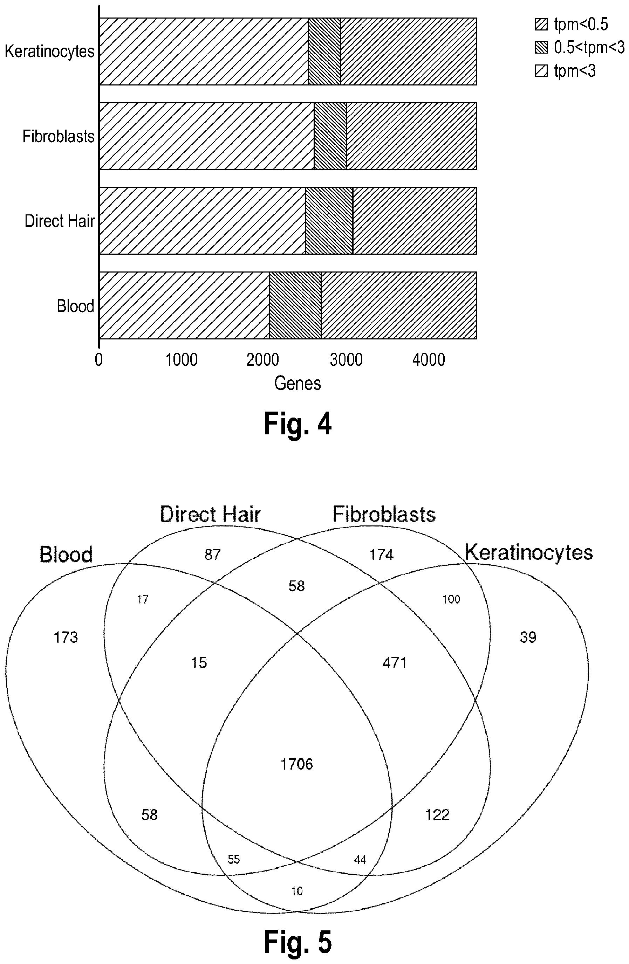

In Fig. 4 the result of the analysis of expression of known disease genes in hair-based keratinocyte cultures and fresh hair roots is shown, compared to frequently used sample types for clinical diagnostics: blood, skin punch biopsy based fibroblast culture. Expression values are measured by normalized read count (TPM = transcripts per million sequenced reads). Gene expression is reliably detectable above 0.5 tpm (yellow), while detection of splice-variants and allele-specific expression requires higher expression values above 3 tpm (green). Blood shows the largest number of diagnostically inaccessible genes (red, tpm < 0.5), while fresh hair shows the least inaccessible genes. Of 4237 OMIM disease genes 877 (21 %) can be measured with high confidence (green, tpm > 3) in keratinocytes or hair roots but not in blood.

### Comparison of OMIM disease genes expressed at confidently detectable levels for diagnostic analysis

The inventors have compared OMIM disease genes expressed at confidently detectable levels for diagnostic analysis (>3tpm) in blood, fibroblasts, fresh hair roots and hair-based keratinocytes. The result is depicted in Fig. 5. A total of 4237 OMIM disease genes were considered, of which 1109 are not reliably detectable in any tissue. Of the remaining 3129 genes the expression of 248 genes (8 %) can only be confidentially measured in hair roots (fresh or cultured). 877 genes (28 %) are not detectable in blood but in hair roots. 174 (5.5 %) of genes can only be confidentially measured in fibroblasts. 94.5 % of the overall detectable disease genes can be diagnostically investigated using a combination of non-invasive blood and hair sampling.

### Similarity of gene expression patterns in different tissues

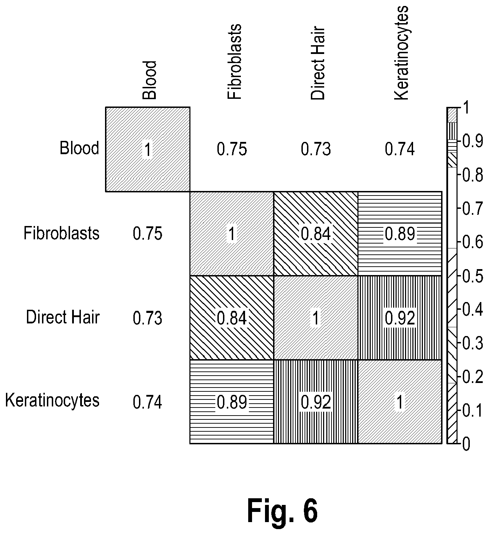

The inventors have analyzed the similarity of gene expression patterns in different tissues. The outcome is illustrated in Fig. 6. Gene expression in fresh hair roots and keratinocytes is more similar to fibroblasts than to blood. This makes hair roots a non-invasive sampling alternative to fibroblasts, which are typically obtained by skin punch-biopsy.

### Keratinocytes are the best sampling material for Nanopore-based transcriptome analysis

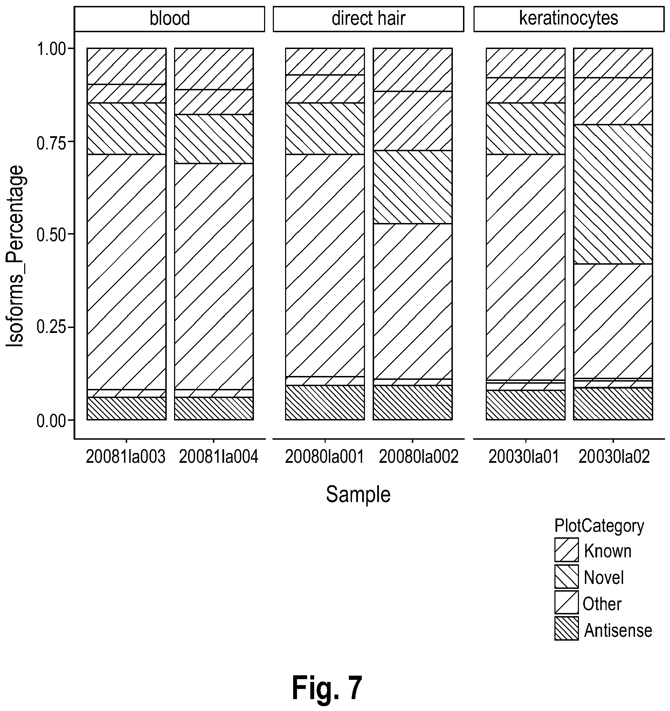

The inventors have examined which sampling material is best for Nanopore-based transcriptome analysis. The result is shown in Fig. 7. Accordingly, keratinocytes are the best sampling material. Up to 70 % of sequenced transcripts map to know genes using only known splice junctions (green) or novel splice-junctions. In comparison, only 30 of sequenced transcripts map to known genes when using blood samples.

### Example application for hair root derived RNA in clinical diagnostics of a rare disease patient

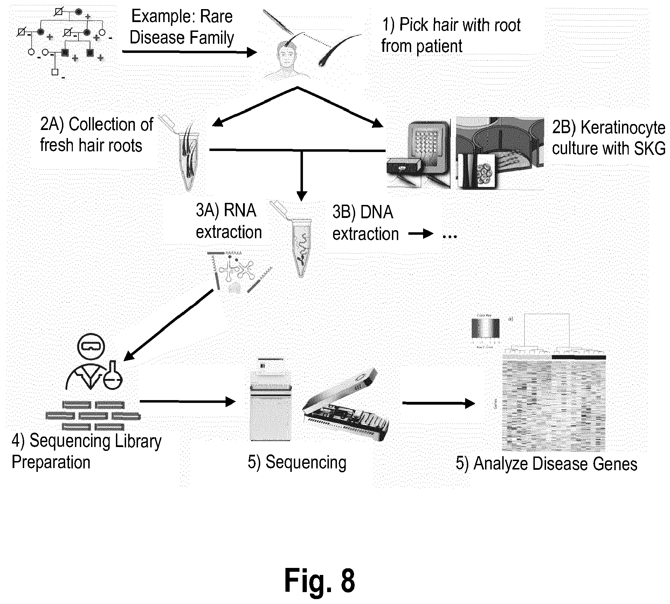

Fig. 8 illustrates an embodiment of the method according to the invention in clinical diagnostics of a rare disease patient. RNA for diagnostics sequencing can be obtained from fresh hair roots or for keratinocyte cultures obtained from a subject suspected of suffering from a rare disease (1, 2A, 2B), followed by RNA (3A) and DNA (3B) extraction, sequencing library preparation (4), sequencing (5) and computational analysis of disease gene expression (5).

### Method variants

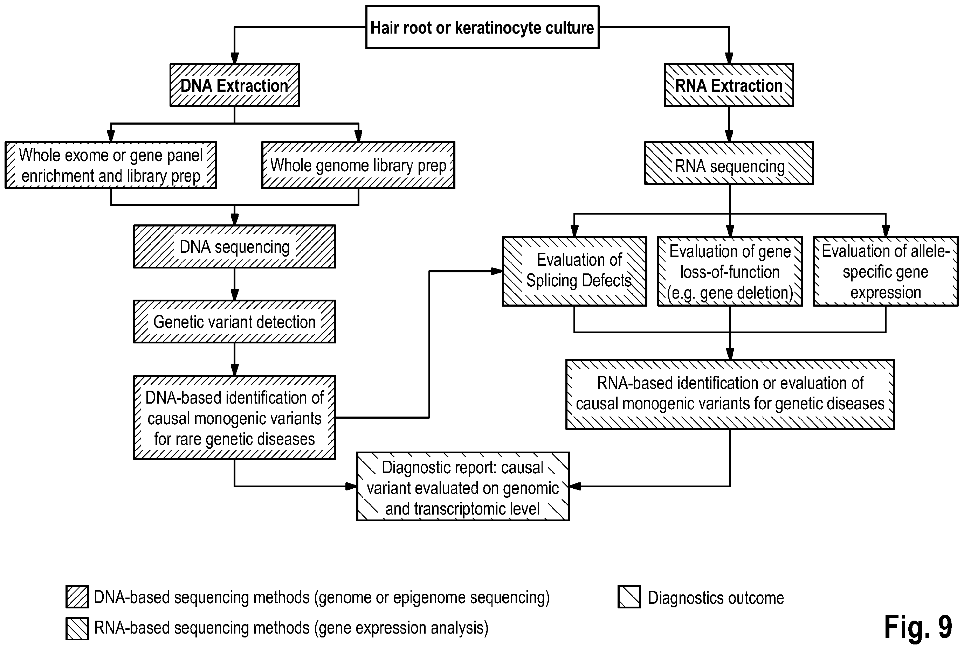

In Figs. 9-12 various variants of the method according to the invention are illustrated. DNA-based sequencing methods (genome or epigenome sequencing) are shown in blue. RNA-based sequencing methods (gene expression analysis) are shown in yellow. Metabolite analysis is shown in red. Diagnostics outcome is shown in green.

In Fig. 9 a flowchart is shown illustrating a variant of the method according to the invention for diagnosis of a (rare) genetic disease. Rare genetic diseases are typically Mendelian and caused by a single variant affecting a disease gene. The diagnostics involves the analysis of DNA (the genome) to identify a causal variant or multiple candidate variants. Subsequently RNA-seq (the transcriptome) can be analyzed to either evaluate candidate causal variants (e.g. splice defect, allele-specific expression, loss-of-function leading to nonsense-mediated decay or deletions, copy-gain and regulatory variants leading to loss or gain of gene expression.

The complete diagnostics can be performed with DNA and RNA extracted from fresh hair roots or keratinocyte cultures. While hair samples have not much advantage for DNA sequencing compared to blood samples, they are almost always a better choice for RNA sequencing and analysis. The reasons are: 1) Quality of RNA extracted from hair roots or keratinocytes is much better than blood-derived RNA; 2) Blood-derived RNA has a very high fraction of hemoglobin (up to 70 %), which has to be depleted (a procedure that further reduces the quality of the RNA), or will take away up to 70 % of the sequencing yield; 3) Many disease genes are not expressed in blood, but in keratinocytes. The inventors estimate that of the known -4000 disease genes 800 can be measured in keratinocytes but not in blood; 4) Hair roots can be preserved much longer than blood samples.

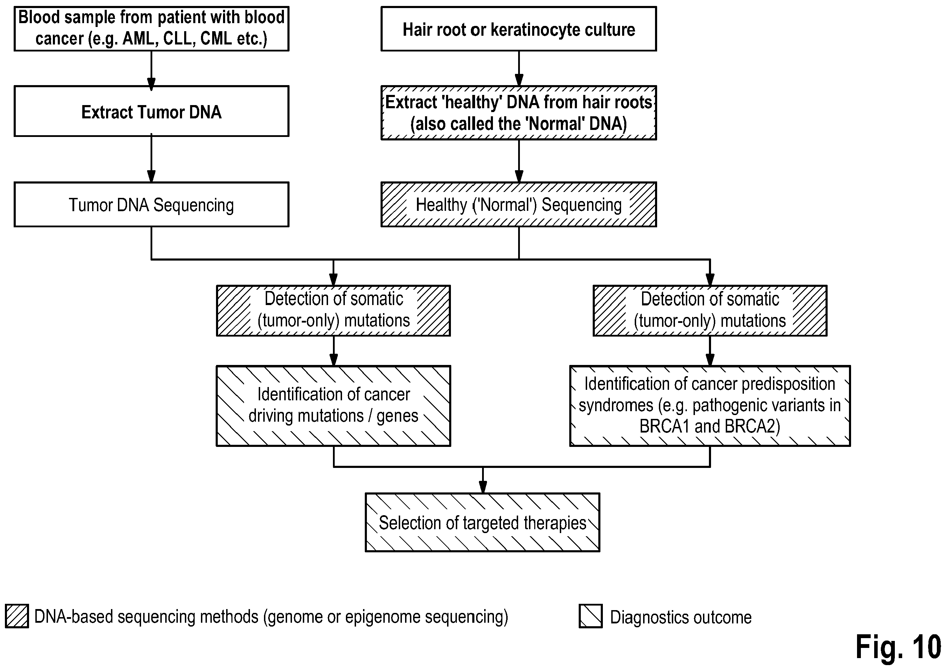

Fig. 10 shows a flowchart illustrating an embodiment of the method according to the invention for diagnosis of blood cancer and selection of targeted therapies. Modern tumor diagnostics by next generation sequencing (NGS) requires the comparison of DNA sequencing data of the tumor samples and of a healthy tissue (the 'normal sample') from the same patient. This tumor-normal sequencing allows for detection of mutations only affecting the tumor (somatic variants), some of which are responsible for tumor proliferation. NGS diagnostics for solid tumors typically uses a blood sample as healthy control. For blood cancers the hair-root derived DNA would be the solution suggested by the inventors for getting healthy ('normal') DNA. At the same time the hair-root DNA can be used to analyze germline (inherited) genetic variants in order to identify cancer risk variants. Note: RNA from hair roots is not useful for tumor diagnostics, as the gene expression in the tumor tissue cannot be compared to gene expression in hair roots.

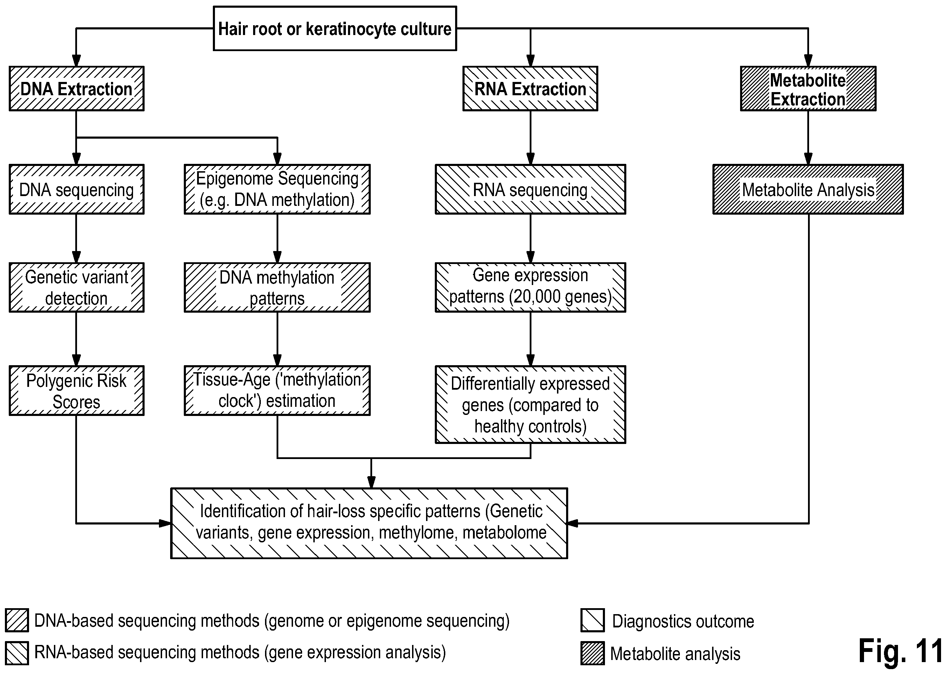

Fig. 11 illustrates in a flowchart an embodiment of the method according to the invention for identifying hair loss specific patterns. Common diseases such as hair loss, often also called complex diseases, are typically multi-genic and are additionally affected by non-genetic factors. Environmental factors, such as nutrition, smoking, alcohol, sports etc., can have a direct or indirect effect on the epigenome or modifications of the DNA, respectively, and the transcriptome or gene expression, as well as on the metabolites in the cells. The epigenome, specifically DNA methylation, holds a footprint of a tissues age and can indicate premature and faster aging of a tissue such as hair roots caused by e.g. genetic predisposition or environmental factors. A combination of genome, epigenome and transcriptome sequencing plus metabolite analysis can be used to identify patterns associated to a common disease such as hair loss. Hair loss, thin hair or bad hair quality could result from genetic predisposition, but also from hormones, environmental factors, metabolites or even drugs such as chemotherapeutics, making a combined analysis of the genome, gene expression, DNA methylation and metabolites in hair roots a promising method for detection of hair-loss specific patterns. The complete procedure can be performed using a small number of fresh hair roots. Genome and epigenome analysis is performed using the extracted DNA. Gene expression analysis is performed using the extracted RNA. Metabolites can be extracted from any tissue and cheap non-sequencing based methods for analysis are available.

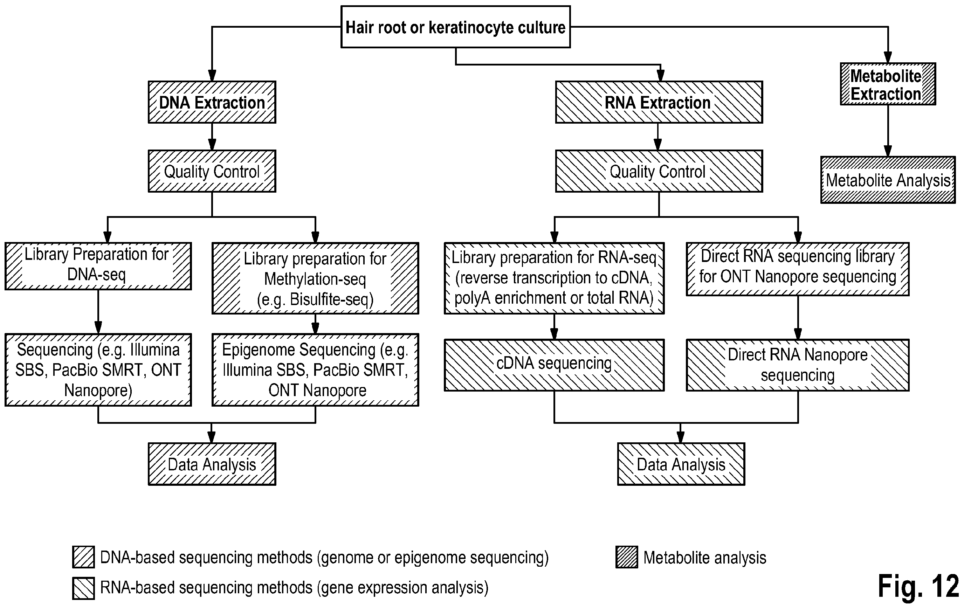

Fig. 12 illustrates in a flowchart an embodiment of the method according to the invention including a generic wet-lab library workflow for the applications described here, including nucleic acid and metabolite extraction, quality control of the extracted biomaterials, sequencing library preparation and sequencing types. Protocols and chemistry for library preparation and sequencing are available from various vendors such as Illumina, NEB, Agilent, Twist, IDT, ONT, Pacific Biosciences etc. Sequencing types include any type of DNA sequencing (whole genome sequencing, whole exome sequencing, gene panel sequencing, amplicon sequencing), epigenome sequencing (e.g. Bisulfite sequencing for DNA methylation analysis, ChIP-seq for DNA-Protein binding or histone modification analysis), RNA sequencing with prior reverse transcription of RNA to cDNA ('cDNA sequencing') or direct RNA sequencing with nanopore technology. Data analysis is sequencing-type specific and has been described above.

## Claims

1. A method for making a genetic determination comprising the following steps:
a) providing a hair root sample from a test individual,
b) isolating nucleic acid from said hair root sample,
c) determining the nucleotide sequence of said nucleic acid to obtain a test nucleotide sequence,
d) comparing said test nucleotide sequence with a reference nucleotide sequence,
e) making a genetic determination depending on the comparison of the test nucleotide sequence with the reference nucleotide sequence.

2. The method of claim 1, wherein said nucleic acid is RNA.

3. The method of claim 1 or 2, wherein said nucleic acid is DNA.

4. The method of any of the preceding claims, wherein in step (c) RNA or/and DNA sequencing is carried out.

5. The method of claim 4, wherein said DNA sequencing comprises epigenome sequencing, preferably to obtain information of methylation of said DNA.

6. The method of any of the preceding claims, wherein after step (a) and before step (b) the following step (a') is carried out:
(a') subjecting said hair root sample to a liquid causing a lysis of the cells and the release of the nucleic acid from the cells, and
wherein in step (b) said nucleic acid is isolated from said liquid.

7. The method of any of the claims 1-5, wherein after step (a) and before step (b) the following step (a") is carried out:
a") cultivating keratinocytes comprised by said hair root sample, and
wherein in step (b) nucleic acid from said cultivated keratinocytes or descendant cells thereof is isolated.

8. The method of any of the preceding claims, wherein before step (a) the following step (a⁰) is carried out:
a⁰) obtaining a hair root sample from a test individual by hair plucking.

9. The method of any of the preceding claims, wherein after step (a) and/or after step (a") the following step (a'") is carried out:
a'") subjecting the hair root sample and/or said cultivated keratinocytes to a metabolome analysis.

10. The method of any of the preceding claims, wherein after step (b) and before step (c) the following step (b') is carried out:
b') preparing a sequencing library based on said nucleic acid, and
wherein in step (d) the nucleotide sequence of said sequencing library is determined to obtain said test nucleotide sequence.

11. The method of any of the preceding claims, wherein said reference nucleotide sequence is a nucleotide sequence of a disease-associated gene, and wherein said genetic determination is a diagnosis of a disease or predisposition to a disease based on the disease-associated gene.

12. The method of claim 11, wherein the reference nucleotide sequence is a nucleotide sequence of tumor DNA of said test individual, preferably of blood tumor DNA, further preferably said blood tumor is selected from the group consisting of acute myeloid leukemia (AML), chronic lymphocytic leukemia (CLL), and chronic myelogenous leukemia (CML).

13. The method of any of the preceding claims, wherein said reference nucleotide sequence is a nucleotide sequence of a baldness-associated gene, and wherein said genetic determination is a determination of reasons of baldness.

14. A kit for making a genetic determination, comprising:
- a container for receiving a hair root sample,
- a nucleic acid sequencing buffer solution, and
- a manual for carrying out the method of any of claims 1-13, and, optionally,
- a cell lysis buffer solution, and, further optionally,
- a keratinocyte culture medium.

15. A use of a hair root sample from a test individual for making a genetic determination based on nucleotide sequencing of nucleic acid isolated from said hair root sample.
